# EUROPÄISCHE PATENTSCHRIFT

(11) **EP 1 993 632 B1**
(45) Veröffentlichungstag und Bekanntmachung des Hinweises auf die Patenterteilung: **10.04.2013**
(21) Anmeldenummer: 06829419.8
(22) Anmeldetag: 08.12.2006
(51) Int. Cl.: A61M 1/36, A61M 5/14, A61M 39/28

(54) **VORRICHTUNG UND VERFAHREN ZUR ÜBERWACHUNG EINES ZUGANGES ZU EINEM PATIENTEN, INSBESONDERE EINES GEFÄSSZUGANGES BEI EINER EXTRAKORPORALEN BLUTBEHANDLUNG**
DEVICE AND METHOD FOR MONITORING ACCESS TO A PATIENT, IN PARTICULAR ACCESS TO VESSELS DURING EXTRACORPOREAL BLOOD TREATMENT
DISPOSITIF ET PROCEDE DE SURVEILLANCE DE L'ACCES À UN PATIENT, EN PARTICULIER L'ACCÈS À DES VAISSEAUX LORS D'UN TRAITEMENT DU SANG EXTRA-CORPOREL

(30) Priorität: 11.03.2006 DE 102006011313
(43) Veröffentlichungstag der Anmeldung: 26.11.2008
(60) Teilanmeldung: 11004023.5
(73) Patentinhaber: Fresenius Medical Care Deutschland GmbH, 61352 Bad Homburg v.d.H. (DE)
(72) Erfinder: KOPPERSCHMIDT, Pascal, 97456 Dittelbrunn (DE)
(74) Vertreter: Oppermann, Frank
(86) Internationale Anmeldenummer: PCT/EP2006/011810
(87) Internationale Veröffentlichungsnummer: WO 2007/104350

(56) Entgegenhaltungen:
- WO-A-2004/020038
- DE-A1- 19 959 965
- FR-A- 1 306 369
- GB-A- 2 046 095
- US-A1- 4 406 042
- US-A1- 5 309 604
- US-A1- 6 113 577
- US-A1- 2001 007 930

## Beschreibung

Die Erfindung bezieht sich auf eine Vorrichtung zur Überwachung eines Zuganges zu einem Patienten für eine Einrichtung, mit der über eine Schlauchleitung eine Flüssigkeit zu dem Patienten geführt oder von dem Patienten abgeführt wird, insbesondere zur Überwachung des Gefäßzuganges bei einer extrakorporalen Blutbehandlung, bei der Blut eines Patienten über eine venöse Schlauchleitung, die eine venöse Punktionskanüle oder -nadel aufweist, dem Patienten zugeführt und über eine arterielle Schlauchleitung, die eine arterielle Punktionskanüle oder -nadel aufweist, von dem Patienten abgeführt wird. Darüber hinaus betrifft die. Erfindung eine Blutbehandlungsvorrichtung mit einem extrakorporalen Blutkreislauf, die über eine Vorrichtung zur Überwachung des arteriellen und/oder venösen Gefäßzuganges verfügt. Des weiteren betrifft die Erfindung ein Verfahren zur Überwachung eines Patientenzuganges.

Auf dem Gebiet der Medizintechnik sind eine Vielzahl von Einrichtungen bekannt, mit denen über eine Schlauchleitung einem Patienten Flüssigkeiten zugeführt oder Flüssigkeiten von dem Patienten abgeführt werden können. Dabei erfolgt der Zugang zu dem Patienten im allgemeinen mit einem Katheter zum Einführen in Körperorgane oder einer Kanüle oder Nadel zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachen.

Ein Anwendungsfall mit besonders hohen Anforderungen an die Sicherheit des Gefäßzuganges stellt die extrakorporale Blutbehandlung dar, bei der über eine arterielle Schlauchleitung, die eine arterielle Punktionskanüle aufweist, Blut von dem Patienten abgeführt, das Blut durch einen Dialysator geleitet wird und über eine venöse Blutleitung, die eine venöse Punktionskanüle aufweist, dem Patienten wieder zugeführt wird. Dabei besteht trotz regelmäßiger Überwachung des Patientenzugangs durch das Krankenhauspersonal grundsätzlich die Gefahr, dass die venöse Punktionskanüle unbemerkt aus dem Blutgefäß des Patienten herausrutscht. Während ein Herausrutschen der arteriellen Kanüle mit einem Ansaugen von Luft in die arterielle Schlauchleitung verbunden ist, das aufgrund einer maschinenseitigen Luftdetektion zu einem visuellen und/oder optischen Alarm und zur Unterbrechung der Behandlung führt, ist das Herausrutschen der venösen Kanüle und der damit gefürchtete Freifluss des Bluts in die Umgebung nicht ohne weiteres zu detektieren. Wenn das Herausrutschen der venösen Kanüle aber nicht sofort erkannt wird, kann der Patient verbluten.

Zur Lösung dieses Problems sind im Stand der Technik eine Vielzahl unterschiedlicher Vorrichtungen bekannt. Einige dieser Vorrichtungen greifen auf standardmäßig in den Blutbehandlungsmaschinen vorhandene Sicherheitsvorrichtungen zurück und lösen bei einem nicht ordnungsgemäßen Gefäßzugang eine sofortige Unterbrechung des extrakorporalen Blutkreislaufs aus. Die standardmäßig in den Behandlungsmaschinen vorhandenen Sicherheitsvorrichtungen basieren im allgemeinen auf einer Überwachung des Drucks im extrakorporalen Blutkreislauf. In der Praxis hat sich jedoch gezeigt, dass allein mit einer Überwachung des Drucks im extrakorporalen Blutkreislauf das Herausrutschen insbesondere der venösen Punktionskanüle nicht mit der ausreichenden Sicherheit erkannt werden kann. Einige bekannte Sicherheitsvorrichtungen haben zwar eine ausreichende Sensitivität, sie reagieren aber sehr empfindlich auf Lageänderungen des Patienten, was oft zu Fehlalarmen führt. Nachteilig ist auch, das sich die bestehenden Blutbehandlungsvorrichtungen nicht ohne weiteres mit den bekannten Überwachungsvorrichtungen nachrüsten lassen, sondern die Nachrüstung einen aufwendigen und kostenintensiven Eingriff in die Behandlungsmaschinen erfordert.

Die DE 44 32 348 C2 beschreibt eine Sicherheitsvorrichtung für eine blut-, wundsekret- oder infusionsführende Schlauchleitung, die auf eine relative Positionsänderung der Schlauchleitung reagiert. Die bekannte Sicherheitseinrichtung verfügt über einen Magneten, der an der Schlauchleitung befestigt ist, und einen Reedkontakt, der an dem Patienten angebracht wird. Wenn an der Schlauchleitung gezogen wird, ändert sich der Abstand zwischen Magnet und Reedkontakt, so dass ein Alarm ausgelöst wird.

Aus der DE 199 53 068 A1 ist eine mechanische Sicherheitsvorrichtung bekannt, die beispielsweise an der Blutleitung einer Dialysemaschine befestigt werden kann. Die bekannte Sicherheitsvorrichtung verfügt über elastisch vorgespannte Klemmbacken, die von einem am Körper des Patienten fixierten Sperrriegel in der geöffneten Position gehalten werden, wobei die Blutleitung zwischen die Klemmbacken eingelegt wird. Eine Lageänderung der Blutleitung führt zu einem Abreißen des Sperrriegels, so dass die Klemmbacken den Schlauch abklemmen. Dies führt zu einem Druckanstieg im Schlauch, der mit den standardmäßig in den bekannten Dialysevorrichtungen vorhandenen Einrichtungen zur Überwachung des Drucks im extrakorporalen Kreislauf detektiert wird. Nachteilig ist, dass die Befestigung der mechanischen Sicherheitsvorrichtung einerseits am Blutschlauch und andererseits am Patienten umständlich ist. Da die Klemmbacken die Schlauchleitung schlagartig abklemmen, ist es nicht möglich, vor der Unterbrechung der Blutbehandlung nur einen Alarm auszulösen. Auch ist aufgrund der auftretenden Kriechvorgänge der Einsatz von kostengünstigen Kunststoffen als Werkstoff bei einer werkseitigen Montage an der Blutleitung problematisch. Grundsätzlich sollten die Klemmbacken nicht unter ständiger Vorspannung stehen, was aber nur dann der Fall ist, wenn die Klemmbacken geschlossen sind. Da der Schlauch bei geschlossenen Klemmbacken aber abgequetscht ist, müssen die Klemmbacken dennoch unter Vorspannung stehen. Wenn die Vorrichtung aus kostengünstigen Kunststoffen gefertigt ist, könnte dies aber in Folge von Kriechvorgangen bereits bei Raumtemperatur mit der Zeit zu einer bleibenden Abnahme der Vorspannung der Klemmbacken und somit zur Unbrauchbarkeit der Vorrichtung führen.

Aus der US-A1-5,309,604 und der DE 199 59 965 A1 beispielsweise sind Befestigungsclips fur flexible Schlauchleitungen bekannt, mit denen sich die Schlauchleitungen in Form einer Schlaufe fixieren lassen. Die bekannten Befestigungsclips sind dafür bestimmt, eine längere Schlauchleitung besser handhaben zu können, wenn nicht die gesamte Schlauchlänge benötigt wird. In diesem Fall soll ein Schlauchsegment der Schlauchleitung mit dem Befestigungselip zu einer Schlaufe fixiert werden.

Die US 2001/0007930 A1 beschreibt eine extrakorporale Blutbehandlungsvorrichtung mit einer Vorrichtung zur Überwachung des Patientenzugangs, bei der der Druck im arteriellen und venösen Zweig des extrakorporalen Blutkreislaufs überwacht wird. Bei einer charakteristischen Änderung des Drucks in den Blutleitungen wird auf einen nicht ordnungsgemäßen Patientenzugang geschlossen.

Der Erfindung liegt die Aufgabe zugrunde, eine einfach zu handhabende, kostengünstig herstellbare und jederzeit nachrüstbare Vorrichtung zu schaffen, die eine sichere Überwachung eines Patientenzuganges erlaubt.

Eine weitere Aufgabe der Erfindung liegt darin, ein Verfahren anzugeben, mit dem auf einfache Weise jederzeit ein Patientenzugang mit hoher Sicherheit überwacht werden kann. Darüber hinaus ist eine Aufgabe der Erfindung, eine extrakorporale Blutbehandlungsvonichtung mit einer Überwachungsvorrichtung für einen Patientenzugang bereitzustellen.

Die Lösung dieser Aufgaben erfolgt erfindungsgemäß mit den Merkmalen der Patentansprüche 1, 16 und 17. Vorteilhafte Ausführungsformen der Erfindung sind Gegenstand der Unteransprüche.

Die erfindungsgemäße Vorrichtung und das erfindungsgemäße Verfahren zur Überwachung eines Patientenzuganges beruhen darauf, dass eine Schlaufe in der flüssigkeitsführenden Schlauchleitung gebildet wird. Es wird davon ausgegangen, dass ein Herausrutschen der Punktionskanüle oder des Katheters auf das Angreifen von Zugkräften an der Schlauchleitung zurückzuführen ist. Wenn die Schlauchleitung auch Zug beansprucht wird, zieht sich die Schlaufe zwangsläufig zu. Dies führt zu einem erhöhten Druckverlust in der Schlauchleitung, der leicht zu detektieren ist.

Die bekannten Blutbehandlungsvorrichtungen verfügen bereits über eine Einrichtung zur Überwachung des Drucks im extrakorporalen Blutkreislauf. Wenn der Druck vorgegebene Grenzen überschreitet, kann die Blutbehandlungsvorrichtung einen Alarm geben und/oder die Blutbehandlung unterbrechen. Die hierzu erforderlichen mechanischen Einrichtungen sind in den bekannten Blutbehandlungsvorrichtungen vorhanden. Daher ist es lediglich erforderlich, die vorgegebenen Grenzwerte für den Druck im extrakorporalen Blutkreislauf auf die verwendete Schlauchleitung abzustimmen.

Von Vorteil ist, dass nicht nur ein fehlerhäfter Patientenzugang leicht erkannt werden kann, sondern dass die Bildung einer Schlaufe gleichsam die Übertragung von Zugkräften auf den Katheter oder die Kanüle schwächen kann. Wenn der Schlauch auf Zug beansprucht wird, zieht sich zunächst die Schlaufe zu, so dass die Zugkräfte nicht sofort auf den Katheter oder die Kanüle übertragen werden. Erst wenn sich die Schlaufe so weit zugezogen hat, dass der Schlauch abknickt, werden die Zugkräfte auf den Katheter oder die Kanüle übertragen. Dann setzt aber schon der Schutzmechanismus ein. Es reicht bereits ein leichter Knick aus, um eine Erhöhung des Druckverlusts in der Schlauchleitung sicher detektieren zu können. Außerdem ist vorteilhaft, dass sich der Staudruck mit zunehmender Strömungsgeschwindigkeit der Flüssigkeit schneller aufbaut. Dies ist insbesondere dann der Fall, wenn die Schlauchleitung aus einem flexiblen Material besteht, das leicht nachgibt.

Wenn die Schlauchlänge entsprechend großzügig bemessen und dem Patienten ein gewisser Bewegungsfreiraum gegeben wird, entstehen nur wenig Fehlalarme, weil im Rahmen üblicher Bewegungen keine oder nur geringe Zugkräfte am Blutschlauch auftreten können. Zudem ist es möglich, die Einrichtung zum Zu- oder Abführen der Flüssigkeit bei einem Störfall nicht sofort abzuschalten, sondern nur einen Alarm auszulösen. Sollte Alarm ausgelöst werden, kann das Krankenhauspersonal den Knick durch Zurückführen der Schlauchleitung in die Schlaufenform beseitigen, ohne dass die Behandlung unterbrochen werden muss.

Die erfindungsgemäße Vorrichtung zur Überwachung eines Gefäßzugangs, mit der eine Einrichtung zum Zu- oder Abführen von Flüssigkeiten zu einem oder von einem Patienten jederzeit nachgerüstet werden kann, weist Mittel zum Fixieren eines Schlauchsegmentes der Schlauchleitung in Form einer Schlaufe auf. Die Erfindung sieht zwei alternative Ausführungsformen vor, die sich dadurch unterscheiden, dass bei der einen Ausführungsform die Mittel zum Fixieren eines Schlauchsegments der Schlauchleitung an einer bestehenden Schlauchleitung angebracht werden können, während bei der anderen Ausführungsform die Mittel zum Fixieren eines Schlauchsegments der Schlauchleitung als einstückiger Bestandteil des Katheters (Punktionsflügel) ausgebildet sind. Wenn der Patientenzugang mit einem Katheter erfolgt, können die Fixierungsmittel auch einstückiger Bestandteil des Katheters sein.

Bei der ersten alternativen Ausführungsform weisen die Fixierungsmittel für das Schlauchsegment ein erstes Befestigungsmittel zum lösbaren Befestigen eines ersten Schlauchsegments und ein zweites Befestigungsmittel zum lösbaren Befestigen eines zweiten Schlauchsegments auf, wobei das erste und zweite Befestigungsmittel miteinander verbunden sind. Die beiden Befestigungsmittel können unterschiedlich ausgebildet sein. Mit dem ersten Befestigungsmittel werden die Fixierungsmittel an einem Schlauchsegment der Schlauchleitung befestigt. Dann wird in der Schlauchleitung von Hand eine Schlaufe gebildet, wobei das Schlauchsegment am Ende der Schlaufe mit dem zweiten Befestigungsmitteln fixiert wird. Dadurch wird verhindert, dass sich die Schlauchleitung wieder in die ursprüngliche Form legt. Wenn die Schlauchleitung zumindest in einem der beiden Befestigungsmittel lose geführt ist, kann sich die Schlaufe bei einer Zugbeanspruchung zunächst so lange zuziehen, bis die Schlauchleitung letztlich abknickt.

Bei einer bevorzugten Ausführungsform weist das erste Befestigungsmittel eine Klammer auf, mit der das Schlauchsegment vorzugsweise klemmend fixierbar ist. Der Durchmesser der Öffnung der Klammer ist vorzugsweise geringfügig kleiner als der Durchmesser der Schlauchleitung, um die Schlauchleitung leicht klemmend zu fixieren, ohne jedoch den Schlauch zusammen zu drücken.

Das zweite Befestigungsmittel weist bei einer weiteren bevorzugten Ausführungsform eine Öse auf, durch die sich Schlauchsegment hindurchziehen lässt. Da das Schlauchsegment in der Öse lose geführt ist, kann sich die Schlaufe der Schlauchleitung unter Zugbeanspruchung zuziehen.

Eine weitere besonders bevorzugte Ausführungsform sieht vor, dass die Schlauchleitung nicht durch die Öse hindurchgezogen wird, sondern sich seitlich in die Öse einlegen lässt. Hierzu wird der Schlauch beim Einlegen in die Öse leicht zusammengequetscht.

Ein Herausrutschen der Schlauchleitung aus der Öse wird dadurch in vorteilhafter Weise verhindert, dass die Öse mit einem Schnappverschluss verschließbar ist.

Es hat sich als vorteilhaft erwiesen, wenn die Schlaufe unmittelbar hinter der Punktionskanüle oder dem Katheter gebildet wird, da die Schlauchleitung an dieser Stelle im Allgemeinen geringeren Beanspruchungen in Form von Lageänderungen des Patienten ausgesetzt ist.

Die alternative Ausführungsform, bei der die Mittel zum Fixieren eines Schlauchsegments der Schlauchleitung einstückiger Bestandteil des Katheters (Punktionsflügel) sind, weist ein Befestigungsmittel zum lösbaren Befestigen eines Schlauchsegments einer Schlauchleitung auf, die an der Punktionskanüle entweder angeschlossen oder anschließbar ist. Die Schlaufe kann einfach dadurch gebildet werden, dass das Schlauchsegment hinter der Punktionskanüle zu einer Schleife geformt und das hinter der Schleife liegende Schlauchsegment mit den Befestigungsmitteln fixiert wird. Vorzugsweise ist das Befestigungsmittel derart ausgebildet, dass das Schlauchsegment lösbar befestigt werden kann. Es ist aber grundsätzlich auch möglich, dass hinter der Punktionskanüle die Schlaufe nicht von Hand gebildet wird, sondern die Schlauchleitung an dem Kathether (Punktionsflügel) dauerhaft eine Schlaufe bildet.

Die Befestigungsmittel für das Schlauchsegment weisen vorzugsweise eine Öse auf, durch die das Schlauchsegment geführt werden kann, so dass sich die Schlaufe bei Zugbeanspruchung zusammenziehen kann. Vorzugsweise ist die Öse zum Einlegen der Schlauchleitung aufspreizbar. Sie kann beispielsweise als Clip oder dgl. für die Schlauchleitung ausgebildet sein.

Im Folgenden werden verschiedene Ausführungsbeispiele der Erfindung unter Bezugnahme auf die Zeichnungen näher erläutert.

Es zeigen:
- Fig. 1: die wesentlichen Komponenten einer Hämodialysevorrichtung zusammen mit der erfindungsgemäßen Vorrichtung zur Überwachung des Patientenzuganges in stark vereinfachter schematischer Darstellung,
- Fig. 2: ein erstes Ausführungsbeispiel der erfindungsgemäßen Vorrichtung in perspektivischer Darstellung,
- Fig. 3: die Vorrichtung von Fig. 2 zusammen mit einer Punktionskanüle und einer Schlauchleitung, wobei die Schlauchleitung nicht auf Zug beansprucht worden ist,
- Fig. 4: die Vorrichtung von Fig. 2, wobei die Schlauchleitung auf Zug beansprucht worden ist,
- Fig. 5: ein zweites Ausführungsbeispiel der erfindungsgemäßen Vorrichtung in perspektivischer Darstellung,
- Fig. 6: ein weiteres Ausführungsbeispiel der erfindungsgemäßen Vorrichtung in perspektivischer Darstellung,
- Fig. 7: die Vorrichtung von Fig. 6 zusammen mit einer Schlauchleitung in perspektivischer Darstellung und
- Fig. 8: eine weitere Ansicht der Vorrichtung von Fig. 6 in perspektivischer Darstellung.

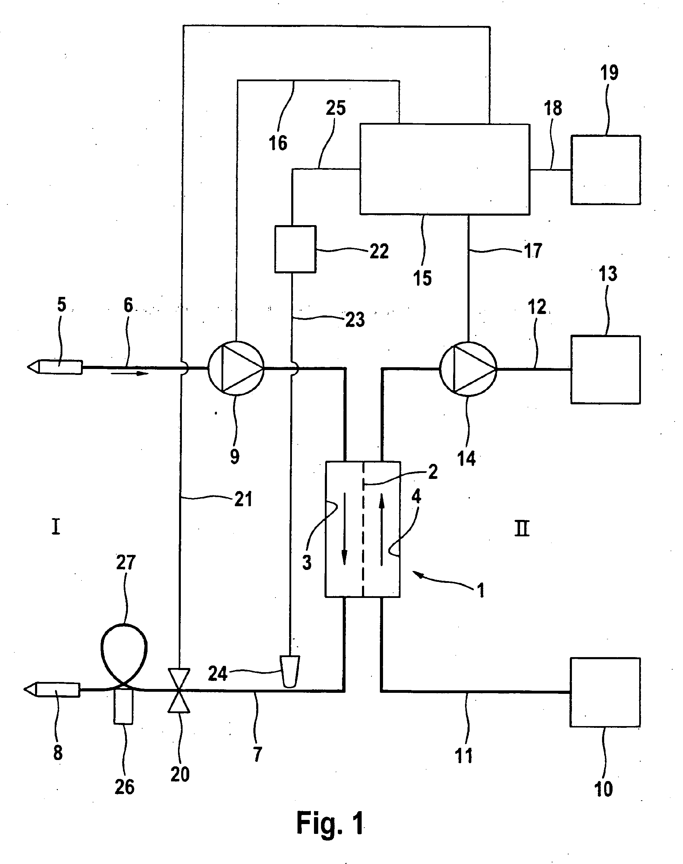

Fig. 1 zeigt die wesentlichen Komponenten einer Hämodialysevorrichtung, die über eine Vorrichtung zur Überwachung des venösen Gefäßzugangs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist. An einer Arterie des Patienten ist mittels einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators 1 geht eine venöse Schlauchleitung 7 ab, die mittels einer venösen Punktionskanüle 8 an einer Vene des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysierflüssigkeitskreislauf II der Hämodialysevorrichtung umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschlossen wird, wenn die venöse Punktionskanüle (Nadel) aus dem Gefäßzugang heraus rutschen sollte. Darüber hinaus stoppt die Steuereinheit 15 nach dem Herausrutschen der Kanüle die Blutpumpe 9.

Zur Überwachung des Drucks in der arteriellen Schlauchleitung weist die Dialysevorrichtung eine Überwachungseinrichtung 22 auf, die über eine Datenleitung 23 mit einem Drucksensor 24 verbunden ist, der den Druck in der venösen Schlauchleitung 7 misst. Die Drucküberwachungseinrichtung 22 kommuniziert mit der zentralen Steuereinheit 15 über eine weitere Datenleitung 25.

Die Vorrichtung zur Überwachung des venösen Gefäßzugangs weist nachfolgend noch im Einzelnen beschriebene Mittel 26 zum Fixieren eines Schlauchsegments der venösen Schlauchleitung 7 in Form einer Schlaufe 27 auf. Die Schlaufe wird stromauf der venösen Punktionskanüle 8 vorzugsweise an einem Abschnitt der Schlauchleitung gebildet, in dem die Schlauchleitung noch am Körper des Patienten, beispielsweise an dessen Unterarm, anliegt.

Während der Dialysebehandlung wird die venöse Schlauchleitung 7 nicht auf Zug beansprucht. Die Drucküberwachungseinrichtung 22 misst einen Druck P in der venösen Schlauchleitung, der innerhalb vorgegebener Grenzen liegt. Die typischen venösen Drücke liegen bei etwa 100 bis 200 mmHg. Es sei angenommen, dass die Druckmesseinrichtung 22 einen venösen Druck von 150 mmHg misst. Bei derartigen Druckverhältnissen wird ein Grenzwertfenster mit einer Breite von 100 mmHg definiert, wobei der untere Grenzwert für den Druck bei 100 mmHg und der obere Grenzwert bei 200 mmHg liegt. Liegt der gemessene Druck ober- bzw. unterhalb der vorgegebenen Grenzen von 100 bzw. 200 mmHg, löst die zentrale Steuereinheit 15 einen optischen und/oder akustischen Alarm aus.

Wenn an der venösen Schlauchleitung 7 stromauf der Schlaufe 27 gezogen wird, besteht die Gefahr, dass die Punktionskanüle 8 aus der Vene des Patienten herausrutscht. Bleibt dies unbemerkt, besteht für den Patienten Lebensgefahr. Der Druckabfall in der venösen Schlauchleitung durch Verlust des Innendrucks im Patientenzugang von etwa 15 bis 25 mmHg kann aber nicht zu einer Unterschreitung des unteren Grenzwertes für den venösen Druck von 100 mmHg führen, so dass ohne die erfindungsgemäße Überwachungsvorrichtung das Herausrutschen der venösen Punktionskanüle nicht erkannt wird.
Da die Schlauchleitung aber oberhalb der Punktionskanüle eine Schlaufe bildet, führt das Herausrutschen der Punktionskanüle zu der Auslösung eines Alarms und/oder die Unterbrechung der Blutbehandlung. Wenn an der venösen Schlauchleitung 7 gezogen wird, zieht sich die Schlaufe 27 zunächst zusammen, so dass sich die Zugspannung zunächst "abfedern" lässt. Eine weitere Zugbeanspruchung führt jedoch dazu, dass sich die Schlaufe 27 so lange zusammenzieht, bis der Schlauch letztlich abknickt. Da sich das Blut an der Knickstelle staut, steigt der venöse Druck in der Schlauchleitung stromauf der Knickstelle. Der Staudruck hängt von dem verbleibenden offenen Querschnitt des abgeknickten Schlauchsegments an der Knickstelle und vom Blutfluss ab. Die Druckmesseinrichtung 22 misst mit dem stromauf der Schlaufe 27 angeordneten Drucksensor 24 den Staudruck vor der Knickstelle. Innerhalb kurzer Zeit, ca. 1 bis 2 Sekunden, steigt der Druck von 150 mmHg aufgrund der Verengung an der Knickstelle stark an, wodurch der obere Grenzwert von 200 mmHg für den venösen Druck überschritten wird. Folglich löst die zentrale Steuereinheit 15 einen Alarm aus und unterbricht die Blutbehandlung durch Schließen der venösen Schlauchklemme 20 und Stoppen der Blutpumpe 9.

Für die Überwachung können verschiedene Grenzwerte unterschiedlicher Höhe definiert werden, so dass darauf geschlossen werden kann, ob die Punktionskanüle aus dem Gefäßzugang herauszurutschen droht oder teilweise oder ganz herausgerutscht ist. Bei Überschreiten der einzelnen Grenzwerte können unterschiedliche Voralarme oder Alarme ausgelöst werden.

Da eine nennenswerte Druckänderung beim Zuziehen der Schlaufe erst relativ spät, d.h. erst mit der Knickbildung auftritt, muss sichergestellt werden, dass die Schlauchleitung tatsächlich abknickt. Es ist vorteilhaft, dies durch eine konstruktive oder strukturelle Anisotropie der Schlauchleitung zu unterstützen, wobei die Form und/oder Beschaffenheit des Materials des Schlauches an der Knickstelle abweichend von der Form und/oder Beschaffenheit des Materials außerhalb der Knickstelle verändert wird. So kann beispielsweise eine Sollknickstelle dadurch geschaffen werden, dass die Schlauchwandung dünner ausgebildet wird oder beispielsweise von der runden Querschnittsform abweicht. So kann die runde Schlauchleitung an der Knickstelle beispielsweise einen elliptischen Querschnitt aufweisen.

Nachfolgend wird ein erstes Ausführungsbeispiel der Mittel beschrieben, mit denen sich auf einfache Weise eine Schlaufe in der Schlauchleitung bilden lässt. Diese Fixierungsmittel können jederzeit an die bestehende Schlauchleitung einer konventionellen Dialysemaschine angebracht werden, die im Allgemeinen bereits über eine Drucküberwachungseinrichtung verfügt, die beim Über- und/oder Unterschreiten von vorgegebenen Grenzwerten reagiert.

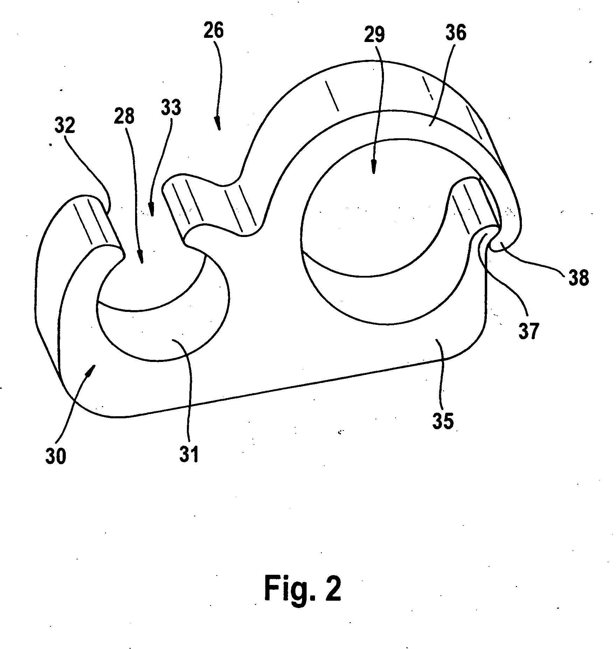

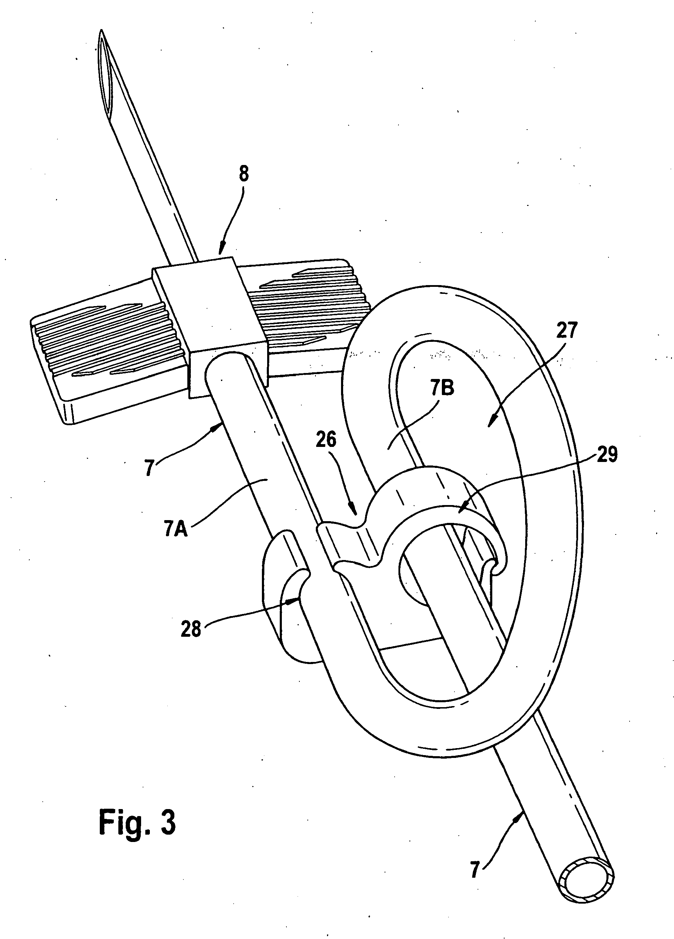

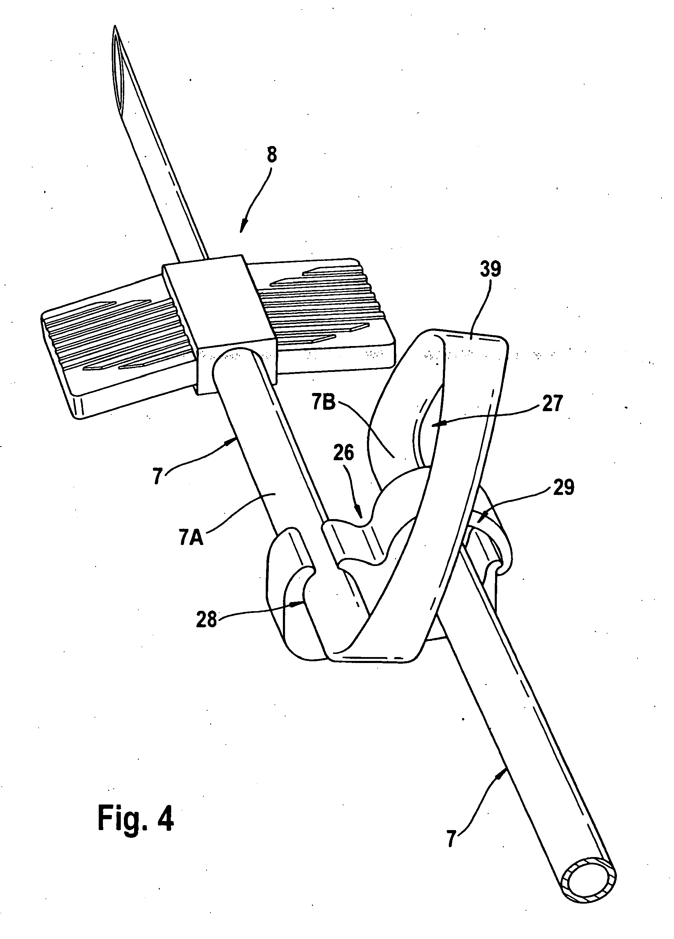

Fig. 2 zeigt die Fixierungsmittel 26 in perspektivischer Darstellung ohne venöse Schlauchleitung, während die Figuren 3 und 4 die Fixierungsmittel 26 zusammen mit der venösen Schlauchleitung zeigen, wobei die Schlauchleitung in Fig. 3 nicht und die Leitung in Fig. 4 auf Zug beansprucht worden ist.

Die Fixierungsmittel 26 verfügen über zwei Befestigungsmittel 28, 29 für die venöse Schlauchleitung 7. Die beiden Befestigungsmittel 28, 29 sind derart angeordnet, dass die damit gehaltenen Schlauchsegmente der Schlauchleitung parallel zueinander verlaufen.

Mit dem ersten Befestigungsmittel 28 werden die Fixierungsmittel 26 an einem Schlauchsegment 7A der venösen Schlauchleitung 7, das stromauf der venösen Punktionskanüle 8 liegt, vorzugsweise unmittelbar vor der Punktionskanüle, angebracht. Das erste Befestigungsmittel 28 ist ein Kunststoff-Spritzgussteil, das in der Art einer Klammer 30 ausgebildet ist, in die das Schlauchsegment 7A eingelegt werden kann. Die Klammer 30 weist eine zentrale Öffnung 31 auf, deren Durchmesser geringfügig kleiner als der Durchmesser des Schlauchsegments 7A ist, so dass das Schlauchsegment von der Klammer klemmend gehalten wird. Die Klammer 30 umschließt das Schlauchsegment 7A bis auf einen oberen Spalt 33, durch den das Schlauchsegment eingeschoben wird, wodurch sich die Klammer leicht aufspreizt.

Zur Befestigung der Fixierungsmittel an der Schlauchleitung können aber anstelle einer Klammer auch zwei parallele Stege vorgesehen sein, deren Abstand geringfügig kleiner als der Durchmesser der Schlauchleitung, so dass die Schlauchleitung zwischen den Stegen leicht klemmend fixiert wird.

An das erste Befestigungsmittel 28 ist ein zweites Befestigungsmittel 29 angeformt, das ein anderes Schlauchsegment 7B der venösen Schlauchleitung 7 hält, wenn die Schlauchleitung eine Schlaufe 27 bildet. Das zweite Befestigungsmittel 29 ist als Öse ausgebildet, die geöffnet oder verschlossen werden kann. Die Öse 29 ist ein Kunststoff-Spritzgussteil mit einem unteren bogenförmigen Teil 35 und einem oberen bogenförmigen Teil 36. Das untere und obere bogenförmige Teil 35, 36 weisen jeweils einen Verschlusshaken 37, 38 auf, die beim Zusammendrücken des unteren und oberen Teils 35, 36 einschnappend ineinander greifen.

Zum Einlegen des Schlauchsegments7B wird das obere bogenförmige Teil 36 nach oben gebogen, so dass das Schlauchsegment in den Spalt zwischen dem oberen und unteren Teil 35, 36 in die Öse 29 eingelegt werden kann. Dann wird die Öse durch Zusammendrücken des oberen und unteren Teils verschlossen, so dass das Schlauchsegment 7B in der Öse unverlierbar gesichert ist. Das Schlauchsegment liegt dabei lose in der Öse, so dass sich die Schlaufe beim Ziehen an der Schlauchleitung zusammenziehen kann. Wenn an der Schlauchleitung 7 gezogen wird, zieht sich die Schlaufe zusammen, bis die Schlauchleitung einknickt. Die Knickstelle ist in Fig. 4 mit dem Bezugszeichen 39 versehen.

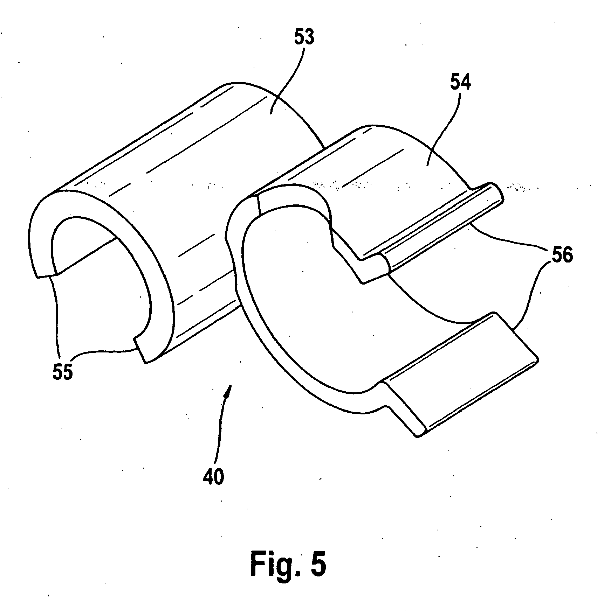

Fig. 5 zeigt in perspektivischer Darstellung ein weiteres Ausführungsbeispiel der Fixierungsmittel 40, die zwei miteinander verbundene Ösen 53, 54 aufweisen, von denen die eine das erste Schlauchsegment 7A und die andere das zweite Schlauchsegment 7B aufnimmt. Beide Ösen können nicht verschlossen werden, sondern halten das jeweilige Schlauchsegment leicht klemmend fest. Während die Endstücke 55 der einen Öse 53 nach innen weisen, zeigen die Endstücke 56 der anderen Öse 54 nach außen. Es können aber auch die Endstücke beider Ösen nach innen bzw. außen zeigen. Die nach außen weisenden Endstücke haben den Vorteil, dass sich die Öse leichter aufspreizen und die Schlauchleitung leichter einlegen lässt.

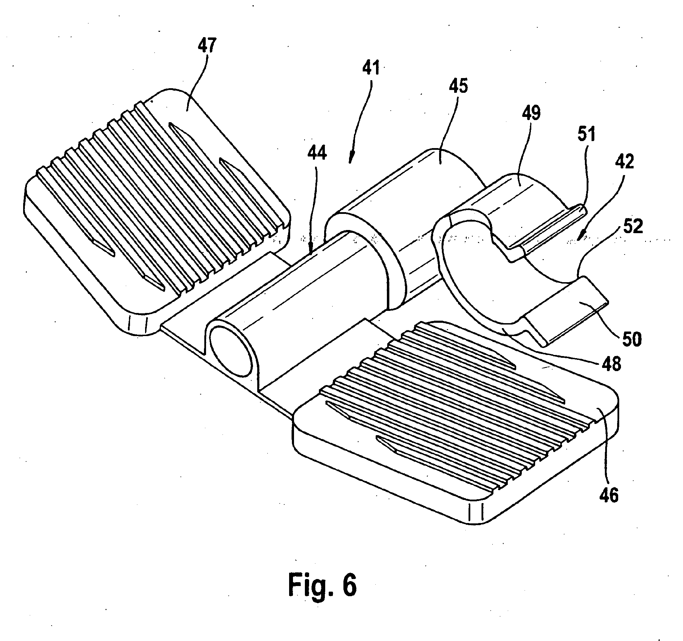

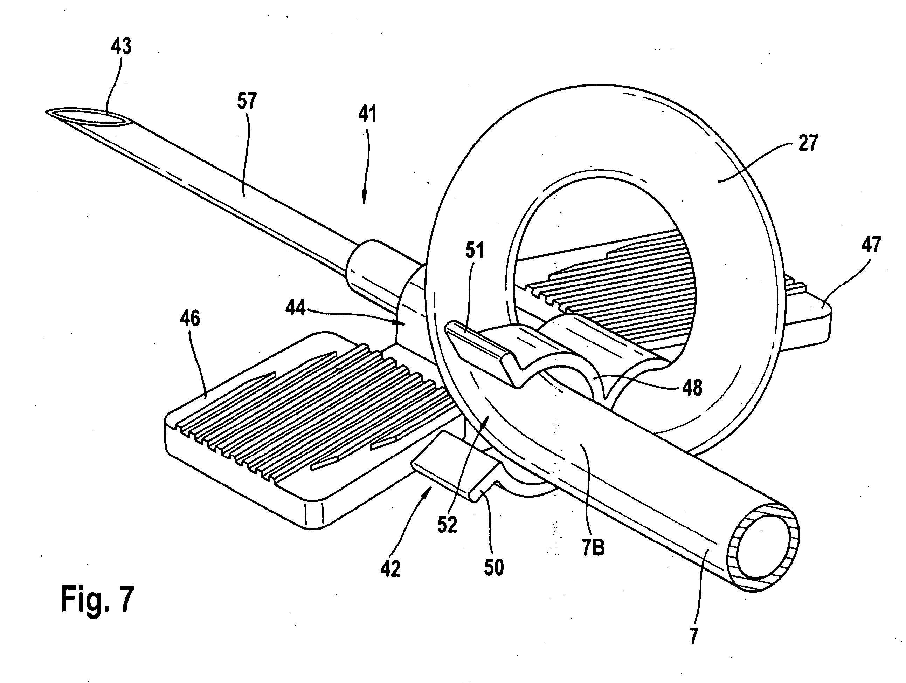

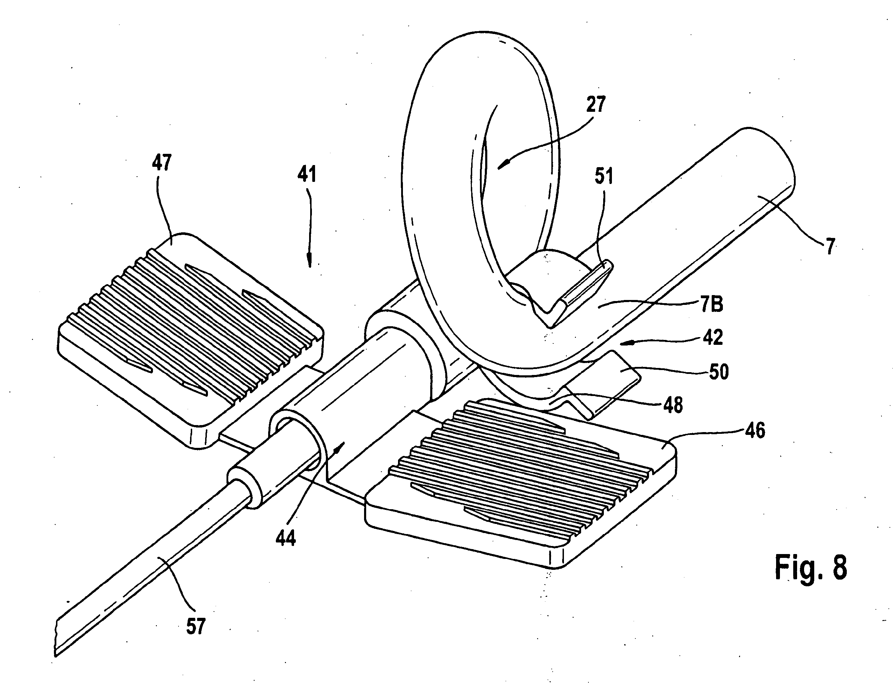

Die Figuren 6 bis 8 zeigen perspektivische Darstellungen aus unterschiedlichen Richtungen einer weiteren alternativen Ausführungsform der Fixierungsmittel 41. Diese Ausführungsform unterscheidet sich von dem unter Bezugnahme auf die Figuren 2 bis 5 beschriebenen Ausführungsbeispielen dadurch, dass die Fixierungsmittel als Punktionsflügel 41 ausgebildet sind. Der erfindungsgemäße Punktionsflügel unterscheidet sich von einher konventionellen Drehflügelkanüle dadurch, dass der Punktionsflügel über ein Befestigungsmittel 42 zur lösbaren Befestigung eines Schlauchsegments 7B einer an der Kanüle angeschlossenen oder anschließbaren Schlauchleitung 7 verfügt. Die Punktionsflügel 41 weist eine Kanülennadel 57 mit einer Anschlifföffnung 43 und ein Schlauchanschlussstück 44 auf, mit dem die Schlauchleitung 7 fest verbunden, beispielsweise verklebt oder verschweißt ist. Zum Anschluss der Schlauchleitung können das Schlauchanschlussstück 44 und das Schlauchleitungsende aber auch mit geeigneten Konnektoren versehen sein. In Fig. 6 ist die Punktionskanüle, die in das Schlauchanschlussstück eingesetzt ist, nicht dargestellt.

Das Schlauchanschlussstück 44 weist einen zylindrischen Körper 45 zur Aufnahme des Schlauchendstücks auf, an dem zu beiden Seiten jeweils ein Flügel 46, 47 angeformt ist. Mit dem zylindrische Körper 45 des Schlauchanschlussstücks 44 ist das Befestigungsmittel 42 verbunden. Das Befestigungsmittel 42 ist eine aufspreizbare Öse, in die das Schlauchsegment 7B der Schlauchleitung 7 nach Bildung der Schlaufe 27 seitlich eingeschoben werden kann. Die Öse besteht aus zwei bogenförmigen Kunststoffstücken 48, 49, die an das Schlauchanschlussstück seitlich angeformt sind und jeweils in einem abgewinkelten Schenkel 50, 51 enden. Nach Bilden der Schlaufe 27 wird die Schlauchleitung 7 durch den Spalt 52 zwischen den beiden Schenkeln 50, 51 seitlich in die Öse eingelegt, in der die Leitung gegen Herausrutschen gesichert ist. Die Schlauchleitung ist wieder lose in der Öse geführt, so dass sich die Schlaufe unter Zugbeanspruchung zusammenziehen und abknicken kann.

## Patentansprüche

1. Vorrichtung zur Überwachung eines Zugangs zu einem Patienten für eine Einrichtung, mit der über eine Schlauchleitung eine Flüssigkeit zu dem Patienten geführt oder von dem Patienten abgeführt wird, insbesondere zur Überwachung des Gefäßzuganges bei einer extrakorporalen Blutbehandlung, bei der Blut eines Patienten über eine venöse Schlauchleitung, die eine venöse Punktionskanüle aufweist, dem Patienten zugeführt und über eine arterielle Schlauchleitung, die eine arterielle Punktionskanüle aufweist, von dem Patienten abgeführt wird,
wobei die Vorrichtung zur Überwachung des Patientenzugangs eine Einrichtung (22) zur Überwachung des Drucks in der Schlauchleitung (6, 7) aufweist, die derart ausgebildet ist, dass bei einer Veränderung des Drucks auf einen nicht ordnungsgemäßen Patientenzugang geschlossen wird,
**dadurch gekennzeichnet,**
**dass** die Vorrichtung zur Überwachung des Patientenzugangs Mittel (26, 41) zum Fixieren eines Schlauchsegments (7A, 7B) der Schlauchleitung (7) in Form einer Schlaufe (27) aufweist, so dass sich bei einer Beanspruchung der Schlauchleitung auf Zug die Schlaufe zusammenziehen kann, und
**dass** die Einrichtung (22) zur Überwachung des Drucks in der Schlauchleitung (6, 7) derart ausgebildet ist, dass auf einen nicht ordnungsgemäßen Patientenzugang bei einer Veränderung des Drucks innerhalb vorgegebener Grenzen aufgrund einer Zugbeanspruchung der Schlauchleitung geschlossen wird.

2. Vorrichtung nach Anspruch 1, **dadurch gekennzeichnet, dass** die Mittel (26) zur Fixierung eines Schlauchsegments (7A, 7B) der Schlauchleitung (7) in Form einer Schlaufe (27) ein erstes Befestigungsmittel (28) zum lösbaren Befestigen eines ersten Schlauchsegments (7A) der Schlauchleitung und ein zweites Befestigungsmittel (29) zum lösbaren Befestigen eines zweiten Schlauchsegments (7B) der Schlauchleitung aufweist, wobei das erste und zweite Befestigungsmittel (28,29) miteinander verbunden sind.

3. Vorrichtung nach Anspruch 2, **dadurch gekennzeichnet, dass** das erste Befestigungsmittel (28) zum Befestigen des ersten Schlauchsegments (7A) eine Klammer (30) aufweist, in die das Schlauchsegment (7A) einlegbar ist.

4. Vorrichtung nach Anspruch 3, **dadurch gekennzeichnet, dass** die Klammer (30) einen Spalt (33) zum Einlegen des Schlauchsegments (7A) aufweist.

5. Vorrichtung nach einem der Ansprüche 2 bis 4, **dadurch gekennzeichnet, dass** das zweite Befestigungsmittel (29) zum lösbaren Befestigen des zweiten Schlauchsegments (7B) eine Öse ist, durch die das Schlauchsegment geführt werden kann.

6. Vorrichtung nach Anspruch 5, **dadurch gekennzeichnet, dass** die Öse (29) zum Einlegen der Schlauchleitung geöffnet werden kann.

7. Vorrichtung nach Anspruch 6, **dadurch gekennzeichnet, dass** die Öse (29) mit einem Schnappverschluss (36) verschließbar ist.

8. Vorrichtung nach Anspruch 1, **dadurch gekennzeichnet, dass** die Mittel (41) zum Fixieren eines Schlauchsegments (7A, 7B) der Schlauchleitung als Katheter ausgebildet sind, an den eine Schlauchleitung (7) angeschlossen oder anschließbar ist.

9. Vorrichtung nach Anspruch 8, **dadurch gekennzeichnet, dass** der Katheter (41) ein Befestigungsmittel (42) zum lösbaren Befestigen eines Schlauchsegments (7B) einer an dem Katheter angeschlossenen oder anschließbaren Schlauchleitung (7) aufweist.

10. Vorrichtung nach Anspruch 9, **dadurch gekennzeichnet, dass** der Katheter (41) ein Schlauchanschlussstück (44) aufweist, wobei das Befestigungsmittel (42) einstückiger Bestandteil des Schlauchanschlussstücks ist.

11. Vorrichtung nach Anspruch 9 oder 10, **dadurch gekennzeichnet, dass** das Befestigungsmittel als Öse (42) ausgebildet ist, durch die das Schlauchsegment (7B) der Schlauchleitung (7) geführt werden kann.

12. Vorrichtung nach Anspruch 11, **dadurch gekennzeichnet, dass** die Öse (42) zum Einlegen des Scblauchsegments (7B) der Schlauchleitung (7) aufspreizbar ist.

13. Vorrichtung nach einem der Ansprüche 1 bis 12 mit einer Schlauchleitung (7) zum Zuführen einer Flüssigkeit zu einem Patienten oder Abführen einer Flüssigkeit von einem Patienten, **dadurch gekennzeichnet, dass** die Schlauchleitung (7) mit einer Sollknickstelle ausgebildet ist.

14. Vorrichtung nach Anspruch 13, **dadurch gekennzeichnet, dass** die Wandung der Schlauchleitung (7) im Bereich der Sollknickstelle eine geringere Stärke aufweist.

15. Vorrichtung nach Anspruch 13, **dadurch gekennzeichnet, dass** die Schlauchleitung mit Ausnahme im Bereich der Sollknickstelle einen runden Querschnitt aufweist, wobei die Schlauchleitung im Bereich der Sollknickstelle einen nicht runden Querschnitt aufweist.

16. Blutbehandlungsvorrichtung mit einem extrakorporalen Blutkreislauf (1), der eine arterielle Schlauchleitung (6) mit einer arteriellen Punktionskanüle (5) und eine venöse Schlauchleitung (7) mit einer venösen Punktionskanüle, (8) aufweist, und mit einer Einrichtung (22) zur Überwachung des Drucks in der arteriellen und/oder venösen Schlauchleitung (6, 7), **dadurch gekennzeichnet, dass** die Blutbehandlungsvorrichtung eine Vorrichtung nach einem der Ansprüche 1 bis 15 aufweist, wobei die Einrichtung (22) zur Überwachung des Drucks in der arteriellen oder venösen Schlauchleitung (6, 7) derart ausgebildet ist, dass bei einer Veränderung des Drucks innerhalb vorgegebener Grenzen aufgrund einer Zugbeanspruchung der arteriellen und/oder venösen Schlauchleitung auf ein Herausrutschen der venösen und/oder arteriellen Punktionskanüle geschlossen wird.

17. Verfahren zum Überwachen eines Zuganges zu einem Patienten zum Zuführen oder Abführen einer Flüssigkeit zu dem Patienten bzw. von dem Patienten über eine Schlauchleitung, wobei der Druck in der Schlauchleitung überwacht wird, **dadurch gekennzeichnet, dass** eine Schlaufe in der Schlauchleitung gebildet wird und dass bei der Veränderung des Drucks innerhalb vorgegebener Grenzen aufgrund einer Zugbeanspruchung der Schlauchleitung auf einen nicht ordnungsgemäßen Patientenzugang geschlossen wird.

## Claims

1. Device for monitoring access to a patient for an apparatus with which a fluid is delivered to the patient or removed from the patient by means of a tubular line, in particular for monitoring vascular access in an extracorporeal blood treatment, in which the blood of a patient is delivered to the patient by means of a venous tubular line which has a venous puncture cannula and is removed from the patient by means of an arterial tubular line which has an arterial puncture cannula,
whereby the device for monitoring the access to a patient comprises an apparatus (22) for monitoring the pressure in the tubular line (6, 7), which is configured so that, when there is a variation of pressure to an improper patient access, it is closed
**characterised in that,**
the device for monitoring access to a patient has means (26, 41) for fixing a tubular segment (7A, 7B) of the tubular line (7) in the form of a loop (27) so that, when the tubular line is subjected to a tractive load, the loop can contract, and
the apparatus (22) for monitoring the pressure in the tubular line (6, 7) is configured so that the tubular line is closed by a tractive load to an improper access to the patient when there is a variation of pressure within the predetermined limits.

2. Device in accordance with claim 1, **characterised in that** the means (26) for fixing a tube segment (7A, 7B) of the tubular line (7) in the form of a loop (27) has a first fastening means (28) for releasably fastening a first tube segment (7A) of the tubular line and a second fastening means (29) for releasably fastening a second tube segment (7B) of the tubular line, whereby the first and second fastening means (28, 29) are connected together.

3. Device in accordance with claim 2, **characterised in that** the first fastening means for fastening the first tube segment (7A) has a clamp (30) into which the tube segment (7A) can be inserted.

4. Device in accordance with claim 3, **characterised in that** the clamp (30) has a gap (33) for insertion of the tube segment (7A).

5. Device in accordance with one of the claims 2 to 4, **characterised in that** the second fastening means (29) for releasably fastening the second tube segment (7B) is an eye, through which the tube segment can be passed.

6. Device in accordance with claim 5, **characterised in that** the eye (29) can be opened for insertion of the tube line.

7. Device in accordance with claim 6, **characterised in that** the eye (29) can be closed with a snap fastening (36).

8. Device in accordance with claim 1, **characterised in that** the means (41) for fixing a tube segment (7A, 7B) of the tubular line is configured as a catheter, to which the tubular line (7) is connected or can be connected.

9. Device in accordance with claim 8, **characterised in that** the catheter (41) has a fastening means (42) for releasably fastening a tube segment (7B) of a tubular line (7) connected or connectable to the catheter.

10. Device in accordance with claim 9, **characterised in that** the catheter (41) has a tube connection piece (44), wherein the fastening means (42) is a one-piece component of the tube connection fitting.

11. Device in accordance with claim 9 or 10, **characterised in that** the fastening means is configured as eye (42), through which the tube segment (7B) of the tubular line (7) can be passed.

12. Device in accordance with claim 11, **characterised in that** the eye (42) can be spread apart for insertion of the tube segment (7B) of the tubular line (7).

13. Device in accordance with one of the claims 1 to 12 with a tubular line (7) for supplying a fluid to a patient or removing a fluid from a patient, **characterised in that** the tubular line (7) is configured with a predetermined kinking point.

14. Device in accordance with claim 13, **characterised in that** the wall of the tubular line (7) has a smaller thickness in the vicinity of the predetermined kinking point.

15. Device in accordance with claim 13, **characterised in that** the tubular line has a circular cross-section with the exception of the region containing the buckling point, such that tubular line has a non-circular cross-section in the region of the buckling point.

16. Blood treatment apparatus , with an extracorporeal blood circuit (I), which comprises an arterial tubular line (6) with an arterial puncture cannula (5) and a venous tubular line (7) with venous puncture cannula (8) and with an apparatus (22) for monitoring the pressure in the arterial and/or venous tube line (6, 7), **characterised in that** the blood treatment device has a device in accordance with one of the claims 1 to 15, wherein the apparatus (22) for monitoring the pressure in the arterial or venous tubular line (6, 7) is configured so that, when there is a variation of pressure within predetermined limits as a result of tractive load, the arterial and/or venous tubular line is closed to the venous and/or arterial puncture cannula slipping out.

17. Method for monitoring access to a patient for delivering or removing a fluid to the patient or from the patient by means of a tubular line, whereby the pressure in the tubular line is monitored, **characterised in that** a loop is formed in the tubular line and that, when there is a variation of pressure within predetermined limits due to a tensile loading, the tubular line is closed to an improper patient access.

## Revendications

1. Dispositif de surveillance d'un accès à un patient pour un appareil avec lequel un liquide est acheminé au patient ou retiré du patient par un tuyau, en particulier pour la surveillance de l'accès vasculaire lors d'un traitement sanguin extracorporel, dans lequel du sang d'un patient est acheminé au patient par un tuyau veineux qui présente une canule de ponction veineuse et est retiré du patient par un tuyau artériel qui présente une canule de ponction artérielle,
le dispositif de surveillance de l'accès au patient présentant un appareil (22) pour la surveillance de la pression dans le tuyau (6, 7) qui est conçu de telle sorte que l'accès non normal au patient soit fermé en cas de modification de la pression
**caractérisé en ce que**
le dispositif de surveillance de l'accès au patient présente des moyens (26, 41) de fixation d'un segment de tube (7A, 7B) du tuyau (7) sous la forme d'une boucle (27) de sorte qu'en cas de contrainte de traction du tuyau la boucle puisse se resserrer, et
l'appareil (22) de surveillance de la pression dans le tuyau (6, 7) est conçu de telle sorte que l'accès non normal au patient soit fermé en cas de modification de la pression dans des limites prédéfinies en raison d'une contrainte de traction.

2. Dispositif selon la revendication 1, **caractérisé en ce que** les moyens (26) de fixation d'un segment de tube (7A, 7B) du tuyau (7) sous la forme d'une boucle (27) présentent un premier moyen de fixation (28) pour fixer de façon amovible un premier segment de tube (7A) du tuyau et un deuxième moyen de fixation (29) pour fixer de façon amovible un deuxième segment de tube (7B) du tuyau, les premier et deuxième moyens de fixation (28, 29) étant reliés l'un à l'autre.

3. Dispositif selon la revendication 2, **caractérisé en ce que** le premier moyen de fixation (28) pour la fixation du premier segment de tube (7A) présente un collier (30) qui peut être placé dans le segment de tube (7A).

4. Dispositif selon la revendication 3, **caractérisé en ce que** le collier (30) présente une fente (33) pour insérer le segment de tube (7A).

5. Dispositif selon l'une des revendications 2 à 4, **caractérisé en ce que** le deuxième moyen de fixation (29) pour fixer de façon amovible le deuxième segment de tube (7B) est un oeillet dans lequel le segment de tube peut être passé.

6. Dispositif selon la revendication 5, **caractérisé en ce que** l'oeillet (29) pour l'introduction du tuyau peut être ouvert.

7. Dispositif selon la revendication 6, **caractérisé en ce que** l'oeillet (29) peut être fermé par une fermeture à cliquet (36).

8. Dispositif selon la revendication 1, **caractérisé en ce que** les moyens (41) de fixation d'un segment de tube (7A, 7B) du tuyau sont conçus comme des cathéters auxquels un tuyau (7) est raccordé ou peut être raccordé.

9. Dispositif selon la revendication 8, **caractérisé en ce que** le cathéter (41) présente un moyen de fixation (42) pour la fixation amovible d'un segment de tube (7B) d'un tuyau (7) raccordé ou pouvant être raccordé au cathéter.

10. Dispositif selon la revendication 9, **caractérisé en ce que** le cathéter (41) présente un embout de raccordement de tube (44), le moyen de fixation (42) étant un composant d'un seul tenant de l'embout de raccordement de tube.

11. Dispositif selon la revendication 9 ou 10, **caractérisé en ce que** le moyen de fixation est conçu comme un oeillet (42) dans lequel le segment de tube (7B) du tuyau (7) peut être passé.

12. Dispositif selon la revendication 11, **caractérisé en ce que** l'oeillet (42) pour l'insertion du segment de tube (7B) du tuyau (7) peut s'élargir.

13. Dispositif selon l'une des revendications 1 à 12, comportant un tuyau (7) pour acheminer un liquide à un patient ou retirer un liquide d'un patient, **caractérisé en ce que** le tuyau (7) est formé d'un site de pliage théorique.

14. Dispositif selon la revendication 13, **caractérisé en ce que** la paroi du tuyau (7) dans la zone du site de pliage théorique présente une résistance inférieure.

15. Dispositif selon la revendication 13, **caractérisé en ce que** le tuyau présente, à l'exception de la zone du site de pliage théorique, une section circulaire, le tuyau présentant, dans la zone du site de pliage théorique, une section non circulaire.

16. Dispositif de traitement sanguin associé à une circulation sanguine extracorporelle (I) qui présente un tuyau artériel (6) comportant une canule de ponction artérielle (5) et un tuyau veineux (7) comportant une canule de ponction veineuse (8) et comportant un appareil (22) pour la surveillance de la pression dans le tuyau artériel et/ou veineux (6, 7), **caractérisé en ce que** le dispositif de traitement sanguin présente un dispositif selon l'une des revendications 1 à 15, l'appareil (22) de surveillance de la pression dans le tuyau artériel ou veineux (6, 7) étant conçu de telle sorte qu'un glissement de la canule de ponction veineuse et/ou artérielle soit fermé en cas de modification de la pression dans une plage de limites prédéfinies en raison d'une contrainte du tuyau artériel et/ou veineux.

17. Procédé de surveillance d'un accès à un patient pour acheminer ou retirer un liquide au patient ou du patient, par un tuyau, la pression dans le tuyau étant surveillée, **caractérisé en ce qu'**une boucle est formée dans le tuyau et **en ce qu'**un accès non normal au patient est fermé en cas de modification de la pression dans des limites prédéfinies en raison d'une contrainte de traction du tuyau.
